# EUROPEAN PATENT APPLICATION

(11) **EP 3 329 878 A1**
(43) Date of publication of application: **06.06.2018**
(21) Application number: 15899709.8
(22) Date of filing: 07.08.2015
(51) Int. Cl.: A61C 8/00, A61C 3/02, A61B 17/17

(54) **MULTI-DRILLING GUIDE DEVICE FOR IMPLANTATION**

(30) Priority: 30.07.2015 KR 20150107720
(71) Applicant: Genoss Co., Ltd., Suwon-si, Gyeonggi-do 16229 (KR)
(72) Inventor: CHUNG, Sung-Min, Suwon-si Gyeonggi-do 16229 (KR); LEE, Sung-Geun, Suwon-si Gyeonggi-do 16229 (KR); SUH, Seung-Woo, Suwon-si Gyeonggi-do 16229 (KR)
(74) Representative: Zardi, Marco
(86) International application number: PCT/KR2015/008304
(87) International publication number: WO 2017/018563

(57) **Abstract**

The present invention relates to a multi drilling guide device for operating an implant, including: a rod extending in a longitudinal direction; a fixing portion combined with one end of the rod and having a drill hole penetrating therethrough; and a guide portion coupled to the rod so as to be movable and rotatable along the rod and having a guide hole.

## Description

### [Technical Field]

The present invention relates to a device capable of guiding drilling upon implanting a fixture of an implant.

### [Background Art]

In general, an implant operation is an operation of placing an artificial dental root on an alveolar bone and fixing an artificial tooth thereon so as to replace a root of a tooth damaged by disease or accident.

The operation of implanting a fixture as the artificial root should be performed more safely and accurately. Therefore, it is necessary to determine an accurate position at which the artificial root is to be implanted before the operation.

However, it has been common practice to implant the fixture at an approximate location determined by the operator's experience or judgment. In such a case, there is a problem that the implant accuracy of the fixture is determined according to the ability of the operator.

In order to solve such a problem, a device having a support having a length corresponding to a fixture identification position and a hole for guiding drilling on both sides of the support has been developed. According to the device, a hole of the support is fixed at a fixture identification location to be able to improve the stability of drilling.

Nevertheless, the above-described device has a problem in that a distance between the holes, that is, a length between the fixtures is fixed as a rod length and therefore a rod having different lengths should be applied when a distance between the fixtures is changed by individual or tooth. In addition, the above-described device also has a problem in that it can not actively cope with a change in an angle between the fixtures as well as the length.

### [Disclosure]

### [Technical Problem]

An object to of the present invention is to provide a multi drilling guide device for operating an implant capable of effectively adjusting an implant location of a fixture upon an implant operation.

### [Technical Solution]

According to another aspect of the present invention, there is provided a multi drilling guide device for operating an implant including: a rod extending in a longitudinal direction; a fixing portion combined with one end of the rod and having a drill hole penetrating therethrough; and a guide portion coupled to the rod so as to be movable and rotatable along the rod and having a guide hole.

The guide portion may be formed to be fixed while being coupled to the rod.

The guide portion may include a penetration coupling hole into which the rod is penetrately inserted; and a fixing screw installed at an opposite side of the guide hole to fix the guide portion to the rod.

The rod may include display scales which are formed to be spaced from each other.

According to another aspect of the present invention, there is provided a multi drilling guide device for operating an implant including: a first rod extending in a longitudinal direction and having a drill hole; a second rod extending in the longitudinal direction and having a guide hole; and a rotation coupling portion connecting the first rod to the second rod so that the first rod and the second rod may move and rotate at different heights.

The rotation coupling portion may include: a first rotation coupling portion coupled to the first rod so that the first rod is penetrately inserted thereinto at a first height; a second rotation coupling portion coupled to the second rod so that the second rod is penetrately inserted thereinto at a second height lower than the first height; and a rotating shaft coupled to the first rotation coupling portion and the second rotation coupling portion.

The rotating shaft may include rotation through holes penetrately formed along the central part thereof.

The first rod may include a support pin coupled to the drill hole.

According to still another aspect of the present invention, there is provided a multi drilling guide device for operating an implant including: a plurality of rods each having drill holes at one end; a plurality of guide portions coupled to the plurality of rods so as to be movable and rotatable along the plurality of rods and having guide holes; and a plurality of rotation coupling portions each coupled to the plurality of guide portions so that the plurality of rods may rotate with respect to the adjacent rods at different heights.

The plurality of rods may include a first rod and a second rod disposed at a first height; and
a third rod disposed at a second height higher than the first height between the first rod and the second rod.

The plurality of guide portions may include: a first guide portion coupled to the first rod; a second guide portion coupled to the second rod; and a third guide portion coupled to the third rod.

The plurality of rotation coupling portions may include: a first rotation coupling portion formed to couple the first guide portion coupled to the first rod to the drill hole of the third rod; and a second rotation coupling portion formed to couple the second guide portion coupled to the second rod to the third guide portion of the third rod.

### [Advantageous Effects]

According to the multi drilling guide device for operating an implant according to the present invention configured as described above, it is possible to effectively adjust the implant position of the fixture.

Further, the guide portion is designed to move or rotate along the rod, so that the fixture can be positioned more precisely.

In addition, the plurality of rotatable rods can be formed without interfering with each other to increase the degree of freedom of the setting of the implant position.

### [BRIEF DESCRIPTION OF THE DRAWINGS]

FIG. 1 is a perspective view illustrating a multi drilling guide device 100 according to an embodiment of the present invention.
FIGS. 2 and 3 are views for explaining a multi drilling guide device 200 having rotatable rods 210 and 230 according to another embodiment of the present invention.
FIGS. 4 and 5 are views for explaining a multi drilling guide device 300 having a plurality of rods 310 which are configured to multi-rotate according to another embodiment of the present invention.

### [Best Mode]

Hereinafter, a multi drilling guide device for operating an implant according to a preferred embodiment of the present invention will be described in detail with reference to the accompanying drawings. In the present specification, same or similar components are denoted by same or similar reference numbers regardless of embodiments, and the description thereof is replaced with the first description.

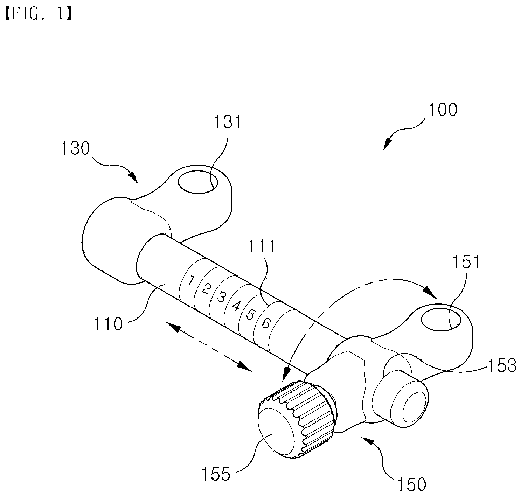

FIG. 1 is a perspective view illustrating a multi drilling guide device 100 according to an embodiment of the present invention.

As shown, the multi drilling guide device 100 may include a rod 110, a fixing portion 130, and a guide portion 150.

The rod 110 may be formed to extend in a longitudinal direction. Specifically, the rod 110 may have a shape like a bar extending in a linear direction. In this embodiment, the rod 110 has a cylindrical shape, but is not limited thereto. The rod 110 may include display scales 111 spaced along an outer surface thereof.

The display scale 111 may be displayed by a number and a line shape having intervals of a predetermined length. Therefore, a user may easily confirm a moving distance of the guide portion 150 through the display scale 111 when the guide portion 150 moves.

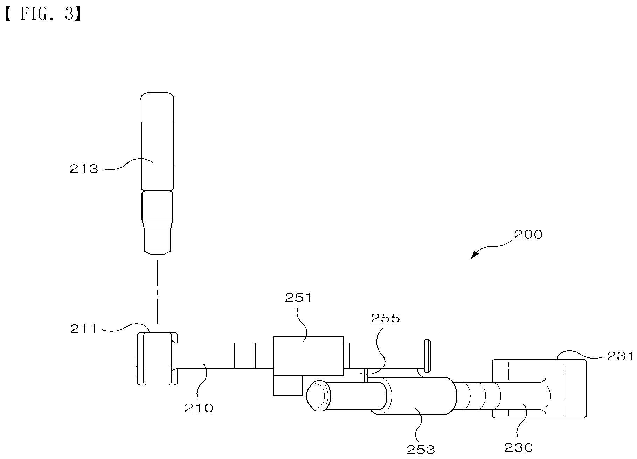

The fixing portion 130 may be coupled to one end of the rod 110. At this time, the fixing portion 130 may be detachably coupled to the rod 110 or may be integrally formed. Further, the fixing portion 130 may extend from the rod 110, and the extending area may be provided with a drill hole 131 penetrating therethrough. In this embodiment, the fixing portion 130 may extend to be perpendicular to the rod 110. The drill hole 131 may penetrate through one surface and the other surface of the fixing portion 130, and a support pin 213 (FIG. 3) or a drill device described later with reference to FIG. 3 may be inserted thereinto.

The guide portion 150 may be coupled to the rod 110 so as to be movable and rotatable along the rod 110. To this end, the guide portion 150 may include a guide hole 151, a penetration coupling hole 153, and a fixing screw 155.

Similar to the drill hole 131, the guide hole 151 may be penetrately formed at a part extending from the guide portion 150.

The penetration coupling hole 153 may be formed so that the rod 110 is penetrately inserted thereinto. Accordingly, the guide portion 150 may move along the rod 110 inserted through the penetration coupling hole 153.

The fixing screw 155 may be provided on an opposite side of the guide hole 151 to fix the guide portion 150 to the rod 110. Specifically, the fixing screw 155 may be formed of a thread passing through an inside of the penetration coupling hole 153. When the fixing screw 155 rotates in one direction, one end of the fixing screw 155 may move while protruding toward an inner space of the penetration coupling hole 153. Therefore, when the rod 110 is inserted into the penetration coupling hole 153 and the fixing screw 155 rotates, one side of the fixing screw 155 may press a surface of the rod 110 to fix the guide portion 150.

Although not shown in detail in the drawings, among the above-described components, the display scale 111 and the penetration coupling hole 153 may have an additional structure. For example, the display scale 111 may be formed as a display groove depressed along the outer surface of the rod 110. Accordingly, an inner surface of the penetration coupling hole 153 may be formed with an elastic catching jaw protruding toward the central part. According to this structure, when the penetration coupling hole 153 is inserted into the rod 110 and moved, the elastic catching jaw may be caught in the display groove. At this time, since the elastic catching jaw has an elastic force, when an external force like a user pushing the guide portion 150 in one direction is generated, the elastic catching jaw may be elastically deformed instantaneously to pass through the display groove. Accordingly, if the guide portion 150 moves along the rod 110, the sequential catching operation may be performed by the display groove.

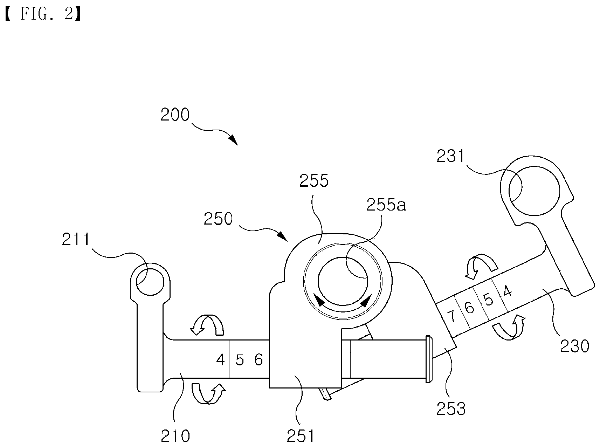

Hereinabove, the structure of the multi drilling guide device was described. The multi drilling guide device having a rotatable rod will be described with reference to FIGS. 2 and 3.

FIGS. 2 and 3 are views for explaining a multi drilling guide device 200 having rotatable rods 210 and 230 according to another embodiment of the present invention.

First, as shown in FIG. 2, the multi drilling guide device 200 may include a first rod 210, a second rod 230, and a rotation coupling portion 250.

The first rod 210 may extend in the longitudinal direction and may be provided with a drill hole 211. The drill hole 211 may be coupled to a support pin 213 as shown in FIG. 3.

The support pin 213 may fix the first rod 210 to the drilled implant position when the plurality of fixtures are implanted. Accordingly, a free end of the first rod 210 may rotate with respect to the support pin 213. The support pin 213 may be detachably attached to the drill hole 211.

The second rod 230 may extend in the longitudinal direction and may have a guide hole 231. In other words, the second rod 230 has substantially the same structure as the first rod 210, and is different from the first rod 210 in that the first rod 210 has the drill hole 211 and the second rod 230 has the guide hole 231.

The rotation coupling portion 250 may be coupled to the first rod 210 and the second rod 230 so that the first rod 210 and the second rod 230 may move and rotate at different heights. To this end, the rotation coupling portion 250 may include a first rotation coupling portion 251, a second rotation coupling portion 253, and a rotating shaft 255.

As in the penetration through hole 153 (FIG. 1), the first rotation coupling portion 251 may be penetrately coupled to the first rod 210 at a first height through the through hole formed therein. Accordingly, the first rod 210 may penetrately inserted into the first rotation coupling portion 251 at the first height.

The second rod 230 may be penetrately coupled to the second rotation coupling portion 253 through the through hole formed inside the second rotation coupling portion 253 at a second height which is lower than the first height.

In addition, each of the rotation coupling portions 251 and 253 may be provided with a fixing screw 155 (FIG. 1) as shown in FIG. 1.

The rotating shaft 255 may be rotatably coupled to the first rotation coupling portion 251 and the second rotation coupling portion 253. As described above, the rotating shaft 255 may be coupled to the first rotation coupling portion 251 and the second rotation coupling portion 253 at different heights so that the first rod 210 and the second rod 230 may rotate at different heights. In other words, the second rotation coupling portion 253 may be coupled to the rotating shaft 255, and the first rotation coupling portion 251 may be coupled to the upper part thereof. Therefore, since the first rotation coupling portion 251 and the second rotation coupling portion 253 are sequentially stacked on and coupled to the rotating shaft 255, they may individually rotate without interfering with each other.

The rotating shaft 255 may be provided with a rotation through hole 255a along the central part thereof. The rotation through hole 255a can be inserted with the support pin 213 or the drill device described above.

According to the multi drilling guide device 200 having such a configuration, the first rod 210 and the second rod 230 may be rotatably coupled to each other about the rotation coupling portion 250. Therefore, the positions of the drill hole 211, the guide hole 231, and the rotation through hole 255a for guiding the implant of the fixture may be easily changed and fixed.

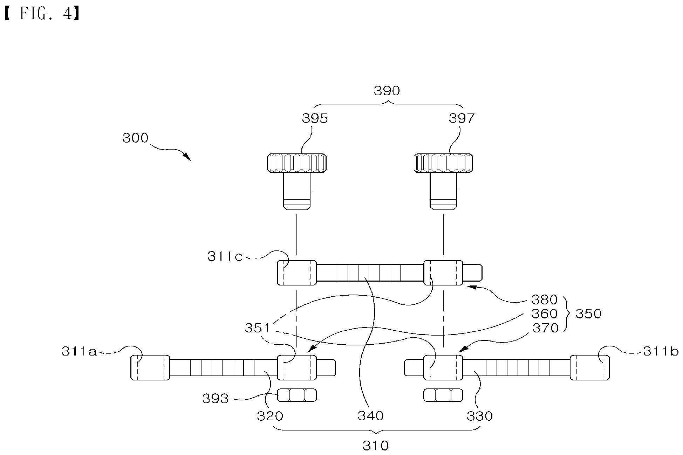

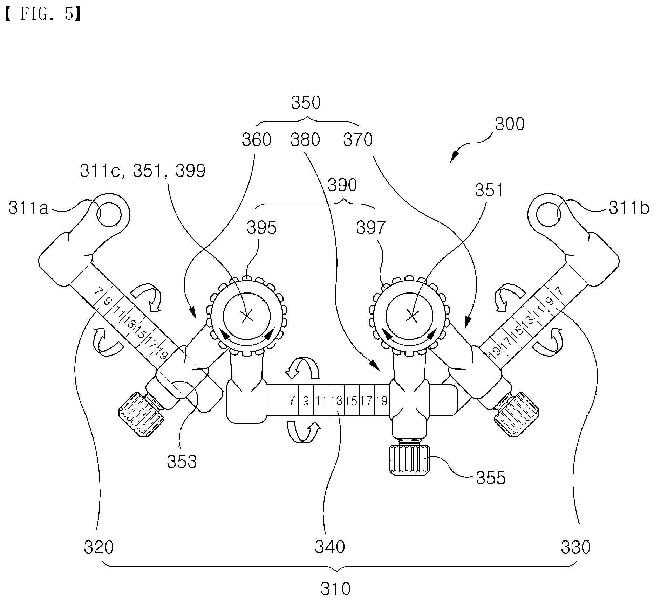

Hereinabove, the multi drilling guide device having the rotatable rod has been described above. The multi drilling guide device which can be multi rotated will be described with reference to FIGS. 4 and 5.

FIGS. 4 and 5 are views for explaining a multi drilling guide device 300 having a plurality of rods 310 which are configured to multi-rotate according to another embodiment of the present invention.

As shown, the multi drilling guide device 300 may include a rod 310, a guide portion 350, and a rotation coupling guide portion 390.

The rod 310 has the same structure as the rod 110 (FIG. 1) of FIG. 1 and may include a first rod 320, a second rod 330, and a third rod 340 each including a first drill hole 311a, a second drill hole 311b, and a third drill hole 311c.

The plurality of rods 310 may be disposed at different locations. Specifically, the first rod 320 and the second rod 330 may be disposed at a first height that is the same height as each other. The third rod 340 may be disposed at a second height that is a height higher than the first height between the first rod 320 and the second rod 330.

Like the guide portion 150 (FIG. 1) of FIG. 1, the guide portion 350 includes a first guide portion 360, a second guide portion 370, and a third guide portion 380 which have a guide hole 351, a penetration coupling hole 353, and a fixing screw 355. Accordingly, the first guide portion 360 may be coupled to the first rod 320, the second guide portion 370 may be coupled to the second rod 330, and the third guide portion 380 may be coupled to the third rod 340.

The rotation coupling portion 390 may be coupled to the plurality of guide portions 350, respectively, so that the plurality of rods 310 can be rotated with respect to the adjacent rods 310 at different heights. The rotation coupling portion 390 may include a first rotation coupling portion 395 and a second rotation coupling portion 397 including a pair of male screws 391 and a female screw 393.

The male screw 391 may be formed of a thread so that a part of an end thereof may be rotatably inserted into the female screw 393. In addition, the male screw 391 may have a diameter enough to be inserted into the guide hole 351 of the guide portion 350 and the drill hole of the rod 310. Accordingly, as shown in FIG. 4, the male screw 391 sequentially penetrates through the guide hole 351 and the drill hole 311 and then may be coupled to the female screw 393 through the protruding end thereof.

Further, the rotation coupling portion 390 may be provided with a rotation through hole 399 which is penetrately formed along a central part thereof. Accordingly, the male screw 391 and the female screw 393 may also be provided with the same rotation through hole 399.

The first rotation coupling portion 395 may rotatably couple the first rod 320 with the third rod 340. Specifically, the first rotation coupling portion 395 may couple the guide hole 351 of the first guide portion 360 coupled to the first rod 320 with a third drill hole 311c of the third rod 340. Accordingly, the third rod 340 is disposed on the first rod 320, and thus when the guide hole 351 of the first guide portion 360 is positioned on the same line as the third drill hole 311c of the third rod 340, the guide hole 351 and the third drill hole 311c may be coupled to each other by the first rotation coupling portion 395.

The second rotation coupling portion 397 may couple the second rod 330 with the third rod 340. In detail, the second rotation coupling portion 397 may couple the second guide portion 370 of the second rod 330 with the third guide portion 380 of the third rod 340. Accordingly, the third rod 340 is disposed on the second rod 330, and thus when the guide hole 351 of the second guide portion 370 is positioned on the same line as the guide hole 351 of the third guide portion 380, the guide hole 351 and the guide hole 351 may be coupled to each other by the second rotation coupling portion 397.

In the multi drilling guide device 300 having the configuration, the plurality of rods 310 may rotate about the first rotation coupling portion 395 and the second rotation coupling portion 397 as an axis. Accordingly, the disposition relationship of the drill hole 311, the guide hole 351, and the rotation through hole 399 that may determine the implant position of the fixture may be more variously applied.

The above-described multi drilling guide device for operating an implant is not limited to the configurations and operations of the embodiments. The above-mentioned exemplary embodiments may also be variously modified through a selective combination of all or some thereof.

## Claims

1. A multi drilling guide device for operating an implant, comprising:
a rod extending in a longitudinal direction;
a fixing portion combined with one end of the rod and having a drill hole penetrating therethrough; and
a guide portion coupled to the rod so as to be movable and rotatable along the rod and having a guide hole.

2. The multi drilling guide device of claim 1, wherein the guide portion is formed to be fixed while being coupled to the rod.

3. The multi drilling guide device of claim 1, wherein the guide portion includes:
a penetration coupling hole into which the rod is penetrately inserted; and
a fixing screw installed at an opposite side of the guide hole to fix the guide portion to the rod.

4. The multi drilling guide device of claim 1, wherein the rod includes display scales which are formed to be spaced from each other.

5. A multi drilling guide device for operating an implant, comprising:
a first rod extending in a longitudinal direction and having a drill hole;
a second rod extending in the longitudinal direction and having a guide hole; and
a rotation coupling portion connecting the first rod to the second rod so that the first rod and the second rod move and rotate at different heights.

6. The multi drilling guide device of claim 5, wherein the rotation coupling portion includes:
a first rotation coupling portion coupled to the first rod so that the first rod is penetrately inserted thereinto at a first height;
a second rotation coupling portion coupled to the second rod so that the second rod is penetrately inserted thereinto at a second height lower than the first height; and
a rotating shaft coupled to the first rotation coupling portion and the second rotation coupling portion.

7. The multi drilling guide device of claim 6, wherein the rotating shaft includes rotation through holes penetrately formed along the central part thereof.

8. The multi drilling guide device of claim 5, wherein the first rod includes a support pin coupled to the drill hole.

9. A multi drilling guide device for operating an implant, comprising:
a plurality of rods each having drill holes at one end;
a plurality of guide portions coupled to the plurality of rods so as to be movable and rotatable along the plurality of rods and having guide holes; and
a plurality of rotation coupling portions each coupled to the plurality of guide portions so that the plurality of rods rotates with respect to the adjacent rods at different heights.

10. The multi drilling guide device of claim 9, wherein the plurality of rods includes:
a first rod and a second rod disposed at a first height; and
a third rod disposed at a second height higher than the first height between the first rod and the second rod.

11. The multi drilling guide device of claim 10, wherein the plurality of guide portions includes:
a first guide portion coupled to the first rod;
a second guide portion coupled to the second rod; and
a third guide portion coupled to the third rod.

12. The multi drilling guide device of claim 11, wherein the plurality of rotation coupling portions includes:
a first rotation coupling portion formed to couple the first guide portion coupled to the first rod to the drill hole of the third rod; and
a second rotation coupling portion formed to couple the second guide portion coupled to the second rod to the third guide portion of the third rod.
